(19) **Europäisches Patentamt**
**European Patent Office**
**Office européen des brevets**

(11) **EP 2 083 252 A1**

(12) **EUROPEAN PATENT APPLICATION**

(43) Date of publication:
**29.07.2009 Bulletin 2009/31**

(51) Int Cl.:
***G01H 1/00*** (2006.01)

(21) Application number: **09151232.7**

(22) Date of filing: **23.01.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **28.01.2008 SE 0800193**

(71) Applicant: **Jönsson, Peter**
**97451 Lulea (SE)**

(72) Inventor: **Jönsson, Peter**
**97451 Lulea (SE)**

(74) Representative: **Holmberg, Nils Anders Patrik**
**Brann AB**
**P.O. Box 171 92**
**104 62 Stockholm (SE)**

(54) **A dosimeter för vibrations**

(57) The present invention relates to an arrangement for calculation and presentation of transferred vibration dose from a handheld machine to the hand of a user, wherein a sensor unit 2 is arranged in the palm 9 of the user. The sensor unit is integrated in a thin textile 1, designed to be worn under a protective glove or similar. The sensor unit 2 may be alternated between different positions in the user's palm 9. The presentation unit 4 communicates wirelessly with an analyzing unit 8 and comprises a fastening device 5 to be fastened around the sleeve of an outdoor garment.

Fig. 3b

**Description**

TECHNICAL FIELD

The invention relates to measurements of vibrations transferred from a machine to a user.

PRIOR ART

Dosimeters for measuring vibrations are generally developed for measuring vibrations when testing new products. Those who benefit from that type of dosimeters are for example development engineers whom, when testing different types of handles, find a basis for judgement of how different types of designs of machines effects transferred energy from a machine to a user. That type of dosimeters typically has a great number of possible adjustments for different cases of measurements. This makes them difficult to use in practice for users lacking education in measurement technology.

There are also examples of dosimeters designed to be used by users lacking thorough knowledge of measurement technology. An example of such a dosimeter is disclosed in WO2006/080880, which is adapted for measurements of vibrations in vehicles.

A special field of measurement technology is measurements of transferred vibrations from handheld machines. Examples of handheld machines are drills, hammer drills, or grinding machines. In that type of measurements it is appropriate to have a sensor arranged between the handle of the tool and the hand of the user. Examples of other machines are such that have a control through which vibrations are transferred to the user. A snowmobile and a compacting machine for road construction are examples of machines having a control.

DE 10119252 discloses an arrangement for measuring hand- and arm vibrations. The user may carry the arrangement on his/her body without that it is hindering the execution of work. However, with DE 10119252 it is not possible to execute measurements on the inner side of a user's hand.

EP 1586875 discloses equipment for the measuring of a cumulative vibration dose transferred to a user's hand-arm. The sensor comprises a calculation unit to calculate the dose of vibrations in a cuff link. The link between the sensor and the calculation unit for calculations is fastened between the user's fingers. Measurement test results are transferred to a docking unit connected to a computer for further analysis, storage and for presentation of the vibration dose. Measurement test results may also be presented numerically on a LCD-display arranged on top of the calculation unit.

A remaining problem is to be able to carry out measurements at any optional point on the inner side of the user's hand. Yet another problem is to present the actual vibration dose at the same time as the user is holding a vibrating machine and is wearing gloves for protection against cold.

OBJECTS AND SUMMARY OF THE INVENTION

An object of the present invention is to solve the above mentioned problems and to provide an arrangement for measuring a cumulative dose of vibrations, which gives reliable measurement test results, and which is easier for the user to put on, and which to a smaller extent interferes with work done with a handheld machine than previously known dosimeters. The present invention makes it possible to perform measurements with a sensor unit placed on the inner side of the user's hand. The arrangement comprises a sensor unit arranged in a thin textile. The textile may be made from an elastic material. Since the sensor is integrated in the textile, the user will not have to fasten the sensor unit, for example between his fingers or between the hand and the tool. The textile, when used, is drawn over the hand, such that the sensor unit is arranged in a position pressed against the machine by the user. The textile is adapted to be carried under a protective glove, a working glove, or similar. The sensor unit and the analysing unit are therefore typically hidden to the user when in use. The arrangement further comprises a presentation unit on which the vibration dose is presented, for example the presentation unit shows a particular symbol if a harmful vibration dose is exceeded. The presentation unit is adapted to be fastened to the user's arm. Since the presentation unit is using wireless communication to communicate with the analyzing unit, the user may use a protective glove, or similar, which covers the sensor unit and the analyzing unit, and still the user can read the actual vibration dose. Thereby, the user, before use, may put on an outdoor garment, for example a quilted jacket, and fasten the presentation unit on the outside of the sleeve of the outdoor garment. The present invention is particularly suitable for use in cold climate.

Further, the sensor unit is movable to different positions on the inner side of the user's hand, which makes the point of measuring being adapted to changes of the starting point for the vibrations. Since the point of measuring is movable and thereby adaptable to the best possible point of measuring, the exposure to vibrations may be measured with high reliability and be presented to the user. The user may, based on the presented vibration dose, adjust the method of application of the machine to minimize the exposure to vibrations. An advantage of the present invention is that it allows the user to increase his/her understanding of how different methods of working effect the transferred vibrations, which after repeated use of the arrangement results in a reduced exposure to vibrations.

SHORT DESCRIPTION OF THE APPENDED DRAWINGS

The present invention is explained more in detail with reference to the appended drawings, where

Figure 1 shows an example of an arrangement according to the invention, wherein a sensor unit is arranged in a thin textile. Further, a presentation unit is shown, which is arranged at an arm of the user.

Figure 2 shows a sensor unit, which is integrated in a thin textile. According to this embodiment, the thin textile is provided as a band. The sensor unit is placed in the user's palm. The band is movable which makes it possible to place the sensor in different positions in the user's palm.

Figure 3a shows the sensor unit and the analyzing unit integrated in a glove-like arrangement. For the purpose of providing the user with good contact with the handle of the machine, the glove may be fingerless. The analyzing unit may be placed in a position at the transition between the hand and the arm. In order to secure the glove-like arrangement, a Velcro tape may be used. The analyzing unit is then advantageously protected by the Velcro tape in the locked state.

Figure 3b shows the analyzing unit hidden and protected by the Velcro tape.

Figure 4 shows an example of a placement of the sensor unit in the user's palm.

Figure 5 shows an illustration of the sensor unit being movable in the glove-like arrangement, for example by means of the textile comprising pocket-like elements designed to have room for the sensor unit.

Figure 6 shows an alternative embodiment, wherein the textile is in the form of a band which is arranged at the fingers of the user.

Figure 7 shows an alternative embodiment, wherein the textile is arranged at one of the fingers of the user. One field of application for this embodiment, is measurement of transferred vibrations to a dentist using a dental drill or a dental polisher.

Figure 8 shows that the sensor unit my be elongated.

DETAILED DESCRIPTION OF THE INVENTION

Compared to previous known dosimeters, the present invention provides a better contact and increased reliability in measurements of vibrations between the hand and the machine, due to the contact surface being arranged at the inner side of the hand where the vibration centre is situated, when using a handheld machine. The arrangement may be used for measurements when using a plurality of different types of machines such as drilling machines, hammer drill, angle grinder, dentist's drill, or chain saw. Further examples of machines are snowmobiles or compacting machines, from which vibrations are transferred through handles or other means of control. The user can easily see the actual vibration dose, and thereby take measures decreasing the dose, even when the user wears working gloves and heavy outdoor garments. Vibration measurements may be carried out at different positions in the hand, for example in the palm, or on a finger. This makes it possible to adjust the position of the sensor unit in order to achieve as correct measurement test results as possible of the vibration exposure.

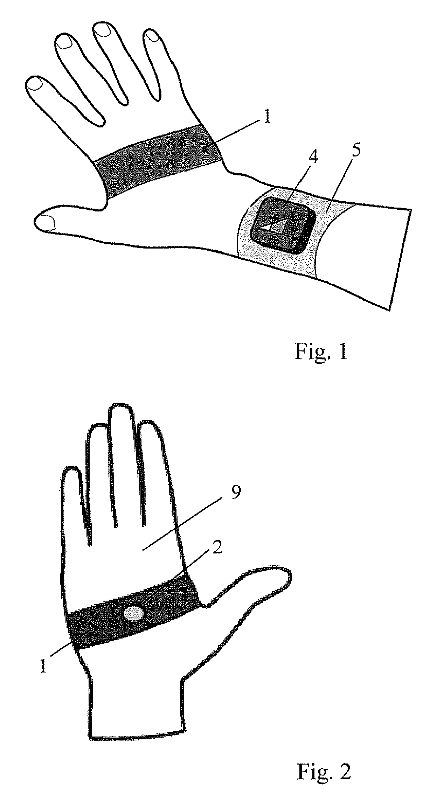

Figure 1 shows an example of an arrangement according to the present invention, wherein a sensor unit 2 is arranged in a thin textile 1. Further, a presentation unit 4 is shown, which is arranged at an arm of a user. The presentation unit 4 is arranged in a fastening device 5 adapted to enclose the arm of the user. An example of such a fastening device 5 is a band or a strap. The presentation unit 4 may also be fastened to a wall, or a tool, wherever visualization is most suitable, i.e. in the visual field of the operator. The textile 1 is according to figure 1 shaped as a band adapted to be drawn over the hand of the user.

Figure 2 shows that the sensor unit 2, when in use, is placed in the palm 9 of the user. Figure 2 illustrates the same example as figure 1, but shows the inner side of the user's hand. According to this embodiment, the thin textile 1 is provided as a band. The sensor unit 2 is placed in the palm of the user. The band is movable, which makes it possible to place the sensor unit 2 at different positions in the palm of the user. The communication is preferably transferred by means of wires 6 to the analyzing unit 8.

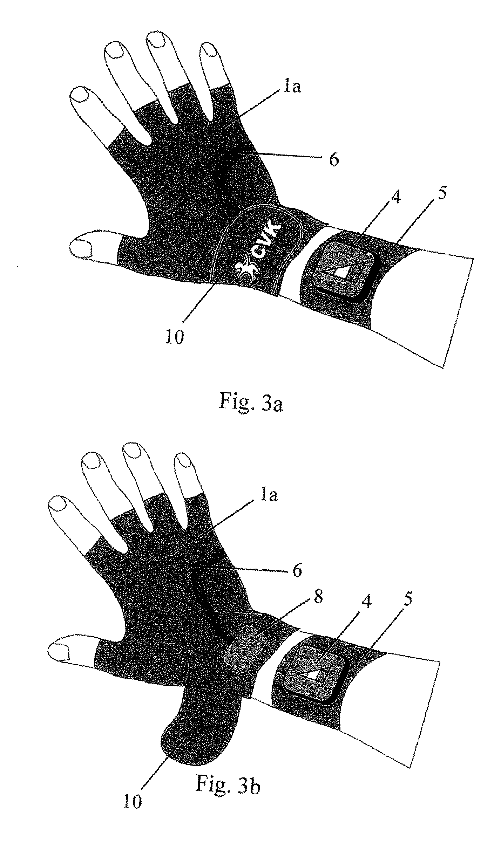

Figure 3a shows the sensor unit 2 and the analyzing unit 8 integrated in a glove-like arrangement 1a. The glove-like arrangement 1a is particularly advantageous since it fixes the sensor unit 2 in a more secure way compared to a band, similar to the one in figure 2. However, an advantage of integrating the sensor unit 2 in a band is that the band, and thereby the sensor unit 2, is easily moved to be adapted to the vibration centre. In order to make the sensor unit 2 movable, the glove-like arrangement 1a comprises a number of fixing elements between which the sensor unit 2 may be alternated. In order to make the user having a good contact with the handle of the machine, the glove 1a may be fingerless. The analyzing unit 8 may be placed at a position at the transition between the hand and the arm, at the wrist. An advantage of this position is that the analyzing unit 8 does not put load on the hand due to its weight, while at the same time it may be integrated in the glove. In order to secure the glove-like arrangement 1a a Velcro tape 10 may be used. The analyzing unit 8 is then advantageously protected by the Velcro tape 10 in the secured state, which can be seen in figure 3b. A further advantage of placing the analyzing unit at the wrist is that the cable, connecting the analyzing unit with the sensor unit is integrated in the glove 1a. The cable pulling according to the embodiment of a glove-like arrangement 1a involves less risk for cable breakdown. This is due to the cable 6 being fixed in the glove 1a when in use. The risk for measurement errors is furthermore decreased since the risk for self-oscillation in the cable is eliminated with a fixed cable.

Figure 3b, shows the analyzing unit 8, hidden and protected by the Velcro tape 10.

The sensor unit typically has a button-like design. Previously known vibration dosimeters for measurement of vibrations at a hand are designed as a sensor unit and an analyzing unit integrated in one structure. This, in contrast to the arrangement wherein the sensor unit 2 is connected with the analyzing unit via a cable 6. The sensor unit 2 has a height which typically is between 3 and 10 mm. This makes it possible to carry the sensor unit 2 under a working glove or similar without interfering with the working operations a user is about to perform. The sensor unit 2 comprises a sensor element and electric circuits, which are adapted to send vibration data to the analyzing unit 4. The sensor element may be a 3-axis accelerometer. The 3-axis accelerometer may be of an analogue type. In that case the dosimeter comprises at least an A/D-converter. The A/D-converter may be integrated in a circuit with a plurality of other components, or mounted as a separate component on a printed circuit card. The 3-axis accelerometer typically supplies measurement quantities like $m/s^2$ or $m/s$. These quantities are converted in the analyzing unit into units relating to frequencies. Furthermore, the sensor unit 2 comprises communication means for transferring measured data to the analyzing unit 8. The analyzing unit 8 is adapted to process vibration data in accordance with standard ISO 5349-1 or other similar measuring methods. A calculation means in the analyzing unit 8 may for example be a microprocessor or a signal processor. The analyzing unit comprises a memory means for storing measured data. The memory means may be built-in or integrated with the calculation means.

According to one embodiment, the analyzing unit 8 comprises means for calculating r.m.s. (root mean square), defined according to the equation below:

$$a_{hw} = \left( \frac{1}{T} \int_0^T a_{hw}{}^2(t)dt \right)^{\frac{1}{2}} \qquad (\text{ms}^{-2}) \qquad (1)$$

wherein T is the time of measurement and $a_{hw}$ is the frequency-weighted acceleration.

This gives the RMS-values for the frequency weighted vibration levels in each direction.

$$a_{hwx}, \ a_{hwy}, \ a_{hwz}$$

The values are thereafter summed up to a vector sum according to the below formula, which gives the total vibration level for vibrations in three directions.

$$a_{hv} = \sqrt{a_{hwx}^2 + a_{hwy}^2 + a_{hwz}^2}$$

The daily vibration exposure is then calculated by:

$$A(8) = a_{hv} \sqrt{\frac{T}{T_0}}$$

The textile 1 may be designed in many different ways. It is an advantage if the textile comprises durable fibres, based on for example acrylate or polyester.

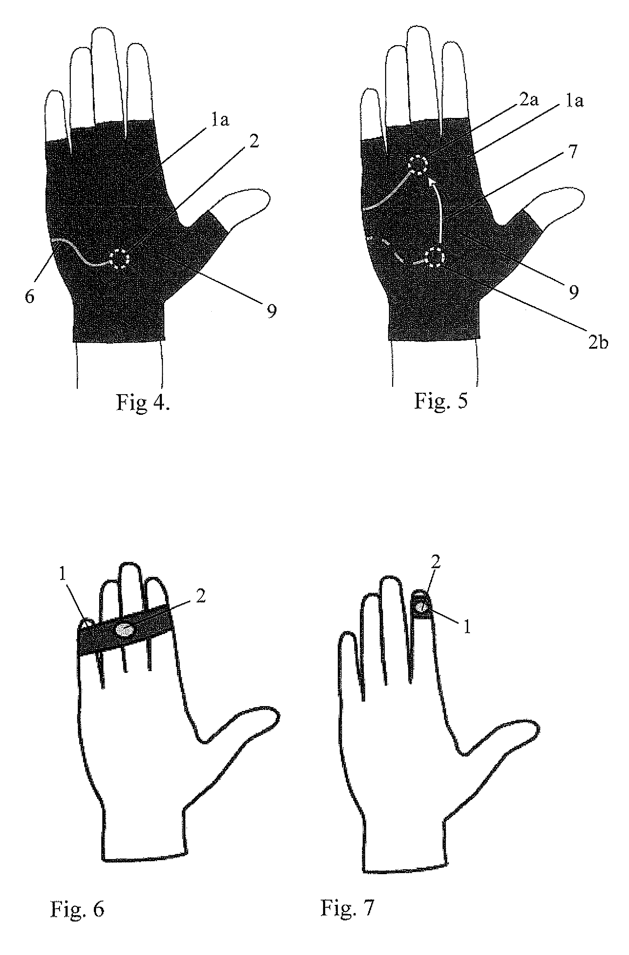

Figure 4 shows an example of how the sensor unit 2 may be placed in the user's palm. The sensor unit may, alternatively, be arranged at some other place, such as at the hand /arm, or in the handle of the machine.

Figure 5 illustrates that the sensor unit 2 is movable, and may be alternated between at least two positions in the glove-like arrangement. The glove-like arrangement 1a comprises at least two fixing elements, between which the sensor unit 2 may be moved. The fixing elements may be designed as small pockets, as Velcro tape or other type of quick locking. Since the sensor unit 2 is movable, the centre of the measuring point is adjustable according to the type of machine which is currently used.

The sensor unit 2 may comprise means for gripping power measurements. Gripping power is an important parameter in transferring vibrations to the hand. According to one embodiment gripping power data is used as basic data for calculation in the analyzing unit 8, for starting or turning off the vibration measurements. Gripping power data may be visualized in the presentation unit 4 to indicate increased vibration load.
In order to adapt to measurement situations wherein high transients occur, it is an advantage if the sensor unit 2 comprises an outer damping coating at the side of the sensor unit 2 that faces the machine. An example of a machine where high transients occur is a hammer drill.

Figure 6 shows an alternative embodiment wherein the textile, in the form of a band, is arranged at the user's fingers.

Figure 7 shows an alternative embodiment wherein the textile is arranged at one of the user's fingers. A field of application for the embodiment is a dentist using a dental drill or a dental polisher.

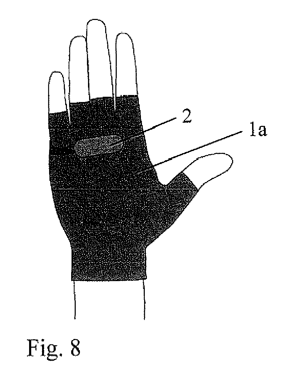

Figure 8 shows that the sensor unit 2 may be elongated.

The present invention is not limited to the above given embodiments, but may be varied in many ways within the scope of the appended claims.

**Claims**

1. An arrangement for measuring a cumulative dose of vibrations transferred to a user of a handheld machine, comprising a sensor unit adapted to be placed in the hand of the user for measuring transferred vibrations to the user's hand;
an analyzing unit comprising means for handling measurement data and calculating a vibration dose;
a presentation unit;
**characterized in that** the sensor unit (2) is arranged in a thin textile (1), whereby the sensor unit (2) and the textile (1) are adapted to be worn under a protective glove, a working glove or similar; the textile (1) is designed such that, when in use, it is drawn over the hand of the user, whereby the sensor unit (2), when measuring, is arranged in a position between the user's palm (9) and the machine; the presentation unit (4) is adapted to communicate wirelessly with the analyzing unit (8) and the presentation unit (4) is arranged in a band- alternatively a strap-like fastening device (5), whereby the presentation unit (4) is adapted to be fastened to the arm of the user.

2. The arrangement according to claim 1 **characterized in that** the sensor unit (2) is button-like and may be alternated between at least two positions in the user's palm (9).

3. The arrangement according to claim 2 **characterized in that** the thin textile (1) is glove-like (1a).

4. The arrangement according to claim 3 **characterized in that** the glove-like textile (1a) comprises at least two fixing elements (2a, 2b), between which the sensor unit (2) may be alternated (7).

5. The arrangement according to claim 2, 3 or 4 **characterized in that** the analyzing unit (8) is integrated in the thin textile (1) in a position which makes it arranged at the upper side of the user's hand or wrist, when in use.

6. The arrangement according to claim 5 **characterized in that** the thin textile (1) comprises a Velcro tape (10), wherein the above lying part is adapted to cover the analyzing unit (8), when the Velcro tape (10) is in a locked state.

7. The arrangement according to any of claims 4, 5 or 6 **characterized in that** the analyzing unit (8) comprises means for calculating the cumulative vibration dose, based on measurements in the sensor unit (2), which comprises a sensor element, which is adapted to perform measurements in at least 3 vector directions.

8. The arrangement according to claim 5, 6 or 7 **characterized in that** the sensor unit (2) comprises an outer damping coating at the side that faces the machine, whereby the arrangement is adapted to a machine which generates high vibration transients.

9. The arrangement according to claim 8 **characterized in that** the sensor unit (2) comprises means for gripping power measurements, and that the analyzing unit (8) is configured to use input data from the means for gripping power measurements, for calculating whether the vibration measurements should be activated or in-activated.

Fig. 1

Fig. 2

1a

6

4 5

CVK

10

Fig. 3a

1a

6

8 4 5

10 Fig. 3b

Fig 4.

Fig. 5

Fig. 6

Fig. 7

Fig. 8

Europäisches
Patentamt

European
Patent Office

Office européen
des brevets

**EUROPEAN SEARCH REPORT**

Application Number

EP 09 15 1232

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DE 101 19 252 A1 (SCHENK KLAUS-THOMAS [DE]; GILLMEISTER FRANK [DE]; MELZIG-THIEL ROMAN []) 21 November 2002 (2002-11-21) * paragraph [0009] - paragraph [0015] * ----- | 1-9 | INV. G01H1/00 |
| Y | US 2002/080031 A1 (MANN JORG [DE] ET AL) 27 June 2002 (2002-06-27) * paragraphs [0013], [0020] - [0023]; figure 2 * ----- | 1-9 | |
| Y | WO 2006/080880 A (JOENSSON PETER [SE]) 3 August 2006 (2006-08-03) * page 14 * ----- | 7 | |
| A | EP 1 586 875 A (MECON LTD [GB]) 19 October 2005 (2005-10-19) * paragraph [0013] * ----- | 1-9 | |
| A | SHELLEY T: "Hands-on sensing wearable vibration dose meter" PANT ENGINEER, vol. 49, no. 4, July 2006 (2006-07), - August 2006 (2006-08) pages 18-19, XP009117338 UK * the whole document * ----- | 1-9 | TECHNICAL FIELDS SEARCHED (IPC)  G01H |
| A | US 4 870 868 A (GASTGEB RAYMOND F [US] ET AL) 3 October 1989 (1989-10-03) * the whole document * ----- | 1-9 | |

The present search report has been drawn up for all claims

| Place of search | Date of completion of the search | Examiner |
|---|---|---|
| Munich | 26 May 2009 | Thomas, Judith |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 09 15 1232

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2009

| Patent document cited in search report | | | Publication date | Patent family member(s) | | Publication date |
|---|---|---|---|---|---|---|
| DE 10119252 | A1 | | 21-11-2002 | NONE | | |
| US 2002080031 | A1 | | 27-06-2002 | NONE | | |
| WO 2006080880 | A | | 03-08-2006 | EP 1842038 A1 | | 10-10-2007 |
| | | | | SE 527616 C2 | | 25-04-2006 |
| | | | | SE 0500191 A | | 25-04-2006 |
| | | | | US 2008134794 A1 | | 12-06-2008 |
| EP 1586875 | A | | 19-10-2005 | GB 2413189 A | | 19-10-2005 |
| US 4870868 | A | | 03-10-1989 | NONE | | |

EPO FORM P0459

For more details about this annex : see Official Journal of the European Patent Office, No. 12/82

**EP 2 083 252 A1**

## REFERENCES CITED IN THE DESCRIPTION

**Patent documents cited in the description**

- WO 2006080880 A
- DE 10119252
- EP 1586875 A